# EUROPEAN PATENT APPLICATION

(11) **EP 1 000 924 A1**
(43) Date of publication of application: **17.05.2000**
(21) Application number: 99953334.2
(22) Date of filing: 20.05.1999
(51) Int. Cl.: C07C 69/54, G03F 7/039, C08F 20/12

(54) **ACID-SENSITIVE COMPOUND AND RESIN COMPOSITION FOR PHOTORESIST**

(30) Priority: 25.05.1998 JP 14353698; 28.08.1998 JP 24406798
(71) Applicant: Daichel Chemical Industries Ltd, Osaka 590-8501 (JP)
(72) Inventor: NAKANO, Tatsuya, Hyogo 670-0094 (JP)
(74) Representative: Grünecker, Kinkeldey, Stockmair & Schwanhäusser Anwaltssozietät
(86) International application number: JP9902637
(87) International publication number: WO9961404

(57) **Abstract**

The photoresist resin composition comprises a polymer containing an acid-responsive compound unit of the following formula (e.g. an adamantane skeleton) and a photoactive acid precursor. R¹ may be an alkyl group having a tertiary carbon atom in the 1-position and the Z ring is a bridged-ring hydrocarbon ring comprising 2 to 4 rings. 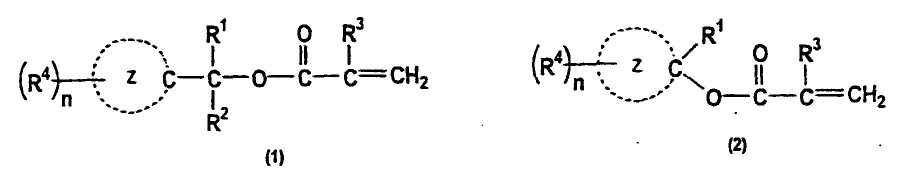 wherein R¹ and R² are the same or different from each other and each represents a hydrogen atom, an alkyl group or a cycloalkyl group; R³ represents a hydrogen atom or a methyl group; R⁴ represents a hydrogen atom, a halogen atom, an alkyl group, an oxygen-containing group, an amino group or an N-substituted amino group; the Z ring represents a monocyclic or polycyclic alicyclic hydrocarbon ring; n represents an integer of not less than 1; with proviso that R⁴ does not concurrently represent a hydrogen atom, and may be different over n occurrences; in formula (1), R¹ and R² may, jointly and together with the adjacent carbon atom, form an alicyclic hydrocarbon ring. The above photoresist resin composition is high in etching resistance, can be solubilized by irradiation, and is capable of providing a finer line pattern.

## Description

### TECHNICAL FIELD

The present invention relates to acid-responsive compounds including adamantane derivatives and to photoresist resin compositions prepared using the acid-responsive compounds. More particularly, the present invention relates to a photoresist resin composition suitable for the formation of a pattern (e.g. minute (fine) processing of semiconductor) using ultraviolet rays or far-ultraviolet rays (inclusive of excimer laser beams) and an acid-responsive compound therefor.

### BACKGROUND ART

The semiconductor integrated circuit is fabricated by a lithographic process which comprises forming a resist thin-layer on a substrate, then forming a latent image pattern by imagewise exposure to light, developing the latent image to form a resist pattern, dry-etching the substrate using the resist pattern as a mask, and removing the resist to provide a designed pattern.

As the resist for semiconductor manufacture, a photosensitive resin composition containing an alkali-soluble novolac resin and a diazonaphtoquinone derivative is known. This resin composition has been used as a positive-acting resist by taking advantage of the phenomenon that on exposure to light the diazonaphthoquinone group is decomposed to give a carboxyl group, whereby the composition which is initially alkali-insoluble is rendered alkali-soluble. There also is known a negative-acting resist, that is a resist which becomes insoluble on exposure to light through a photo-crosslinking reaction in the presence of an azide compound or a photopolymerization reaction in the presence of a photopolymerization initiator.

Meanwhile, in the lithographic technology, the demand for a finer line pattern definition has caused a shift from ultraviolet rays, such as g-line and i-line, to rays of shorter wavelengths, such as far-ultraviolet rays, vacuum ultraviolet rays, excimer laser beams, an electron beam and X-rays.

However, because the resins used contain an aromatic ring, those resists may at times be opaque to light at wavelengths shorter than 200 nm and are not suited (inactive) as compositions for use with an ArF excimer laser which has a wavelength of 193 nm.

As a photoresist suitable for short-wavelength exposure light sources (e.g. ArF excimer laser). Japanese Patent Application Laid-Open No, 73173/1997 discloses a resist material comprising a polymer having a structural unit protected by an alicyclic hydrocarbon group, such as adamantane or norbornane, which is cleaved (eliminated) by an acid to render the material alkali-soluble in combination with an acid precursor. This literature mentions, as the polymer, (1) a copolymer of 2-methyl-2-adamantyl (meth)acrylate and (2) a copolymer of 2-(1-adamantyl)propyl (meth)acrylate, among others. The above polymer having no double bond within its ring structure is transparent (active) to the ArF excimer laser beam and, in semiconductor fine processing, the resistance to plasma gas dry-etching is enhanced.

However, when the above resist comprising the polymer and the acid precursor is used to form a pattern, the tendency toward formation of cracks and peeling of the pattern is increased as the pattern line becomes finer so that it is sometimes impossible to form a pattern of fine line definition.

The present invention, therefore, has for its object to provide an acid-responsive compound having an alicyclic hydrocarbon group (e.g. an adamantane skeleton) and capable of providing an alkali-soluble polymer on exposure to light, thus being useful for the formation of fine-line patterns and a photoresist resin composition containing the compound.

It is another object of the present invention to provide an acid-responsive compound which is high in sensitivity and etching resistance (particularly resistance to dry etching) and instrumental in forming a fine-line resist pattern with good reproducibility and high precision and a photoresist resin composition containing the compound.

It is a still another object of the present invention to provide an acid-responsive compound showing high adhesion to a substrate and useful for forming a fine-line resist pattern with high precision and high reproducibility and a photoresist resin composition containing the compound.

### DISCLOSURE OF INVENTION

The inventors of the present invention did intensive investigations for accomplishing the above objects and found that when a polymer comprising an acid-responsive compound unit having an alicyclic hydrocarbon group and a specific structure is used in combination with a photoactive acid precursor, the alicyclic hydrocarbon group is stably and efficiently eliminated from the polymer by the acid formed from the acid precursor on exposure to light to thereby enable water or alkali development. The present invention has been developed on the basis of the above finding.

The acid-responsive compound according to the present invention, therefore, is represented by the following formula (1) or (2). 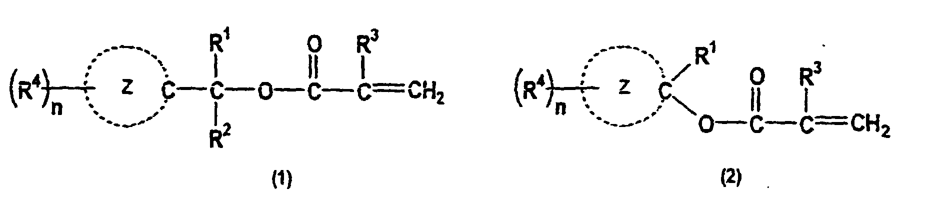 wherein R¹ and R² are the same or different from each other and each represents a hydrogen atom, an alkyl group or a cycloalkyl group; R3 represents a hydrogen atom or a methyl group; R4 represents a hydrogen atom, a halogen atom, an alkyl group, an oxygen-containing group, an amino group or an N-substituted amino group; n represents an integer of not less than 1; with proviso that all R⁴s are not concurrently hydrogen atoms, R⁴ may be vary according to a; the Z ring represents a monocyclic or polycyclic alicyclic hydrocarbon ring; in formula (1),R¹ and R² may, jointly and together with the adjacent carbon atom, form an alicyclic hydrocarbon ring.

In this acid-responsive compound, the ring Z may be a bridged (clossliked) ring-type hydrocarbon ring containing 2 to 4 component rings (e.g. an adamantane ring). Such compounds include adamantane derivatives represented by the following formula (1a) or formula (2a) and the compounds represented by the following formula (2d) or formula (2e). 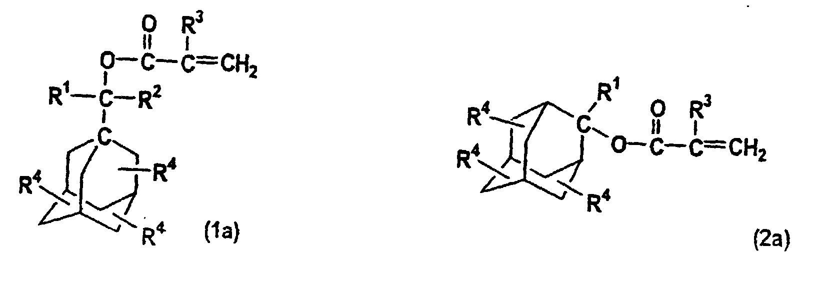 wherein R¹, R² and R³ are as defined above; R⁴s may be the same or different from each other and each represents a hydrogen atom, a halogen atom, an alkyl group, an oxygen-containing group, an amino group or an N-substituted amino group; with proviso that that all of R⁴s are not hydrogen atoms. 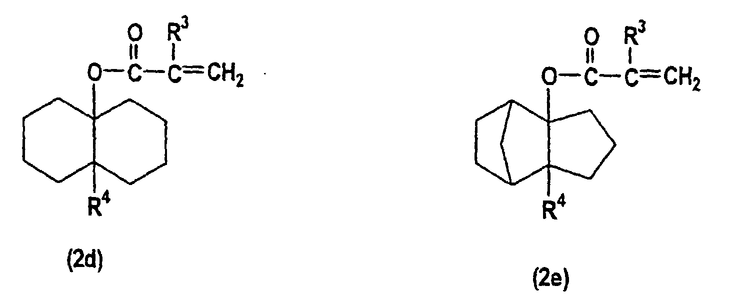 wherein R³ and R⁴ are as defined hereinbefore.

The photoresist resin composition of the present invention can be constituted by a polymer having at least a repeating unit of the following formula (11) or formula (12) and a photoactive acid precursor (a photoactive acid generator). 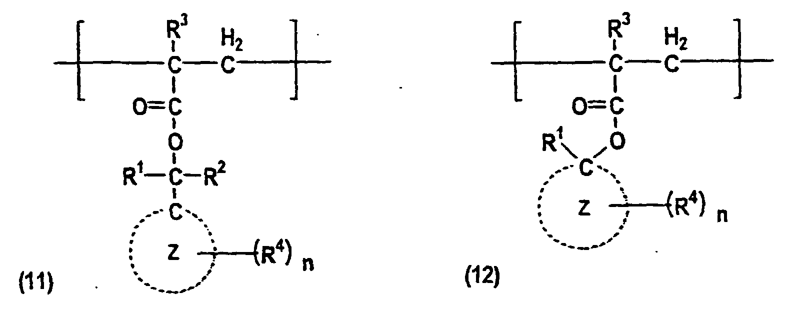 wherein R¹, R², R³, R⁴, Z ring and n are as defined hereinbefore.

In this polymer, the Z ring may be a bridged-ring (bridged ring-type) hydrocarbon ring containing 2 to 4 rings (e.g. an adamantane ring).

The present invention is further directed to a method of forming a pattern which comprises subjecting a layer comprising the photoresist resin composition on a substrate to imagewise exposure to light and developing the exposed coating layer to form a pattern.

### BEST MODE FOR CARRYING OUT THE INVENTION

Referring to the above formulas (1), (2), (1a), (2a), (11) and (12), the alkyl groups represented by R¹ and R² each includes straight-chain or branched-chain alkyl, e.g. C₁₋₁₀ alkyl groups (preferably C₁₋₆ alkyl groups and particularly C₁₋₄ alkyl groups), such as methyl, ethyl, propyl, isopropyl, butyl, isobutyl, s-butyl, and t-butyl, etc.

The branched-chain alkyl group may be an alkyl group containing a tertiary carbon atom. The branched-chain alkyl group having a tertiary carbon atom includes 1-C₁₋₄ alkyl-C₁₋₆ alkyl groups such as isopropyl, isobutyl, 1-methylethyl, isopentyl, 1-methylpropyl(s-butyl), 1-methylbutyl(s-pentyl), s-hexyl, 1-ethylethyl, 1-ethylbutyl, etc. The preferred branched-chain alkyl group R¹ includes alkyl groups having a methine carbon atom in the α-position, particularly 1-C₁₋₂ alkyl-C₁₋₄ alkyl( isopropyl, s-butyl, etc.) groups. Referring, further, to formulas (1), (2), (1a), (2a), (11) and (12), when the Z ring has no substituent, the alkyl group designated by R¹ is a branched-chain alkyl group containing a tertiary carbon atom.

The cycloalkyl group includes C₃₋₁₅ cycloalkyl groups, for example monocyclic C₃₋₁₀ cycloalkyl groups such as cyclopropyl, cyclobutyl, cyclopentyl, cyclohexyl, cyclohexyl, cyclooctyl, and cyclodecyl, and polycyclic C₆₋₁₅ cycloalkyl groups such as perhydronaphthyl (decalyl), adamantyl, methyladamantyl, and dimethyladamantyl.

In the formulas (1), (2), (1a), (2a), (2d), (2e), (11) and (12), R³ represents a hydrogen atom or a methyl group, forming an acryloyl or methacryloyl group.

R¹ and R² may, jointly and together with the adjacent carbon atom, form an alicyclic hydrocarbon ring. This alicyclic hydrocarbon ring includes hydrocarbon rings corresponding to the cycloalkyl group.

The Z ring includes a variety of alicyclic hydrocarbon rings, for example monocyclic hydrocarbon rings and polycyclic hydrocarbon rings (spiro hydrocarbon ring, ring assembly hydrocarbon ring, fused-ring (condensed ring-type) hydrocarbon ring, and bridged-ring (closslinked ring-type) hydrocarbon ring). The monocyclic hydrocarbon ring includes but is not limited to C₄₋₁₀ cycloalkane rings such as cycloheptane, cyclohexane, cyclopentane, cyclooctane, etc. and the spiro hydrocarbon ring includes but is not limited to C₈₋₁₆ hydrocarbon rings such as spiro[4.4]nonane, spiro[4.5]decane, spirobicyclohexane, etc. The ring assembly hydrocarbon ring includes but is not limited to hydrocarbon rings having C₅₋₁₂ cycloalkane rings, such as bicyclohexane ring, biperhydronaphthalene ring, etc. The fused-ring (fused ring-type) hydrocarbon ring includes, for example, fused-ring rings containing 5 to 8-membered cycloalkane component rings such as perhydronaphthalene ring (decalin ring),. perhydroanthracene ring, perhydrophenanthrene ring, perhydroacenaphthene ring, perhydrofluorene ring, perhydroindene ring, perhydrophenalene ring, etc.

The preferred Z ring is a bridged-ring hydrocarbon ring. The bridged-ring hydrocarbon ring includes but is not limited to bicyclic hydrocarbon rings such as pinane, bornane, norpinane, norbornane, etc.; tricyclic hydrocarbon rings such as homobrendane, adamantane, tricyclo[5.2.1.0^{2,6}]decane, tricyclo[4.3.1.1^{2,5}]undecane, etc.; tetracyclic hydrocarbon rings such as tetracyclo[4.4.0.1^{2,5}.1^{7,10}]dodecane, perhydro-1,4-methano-5,8-methanonaphthalene, etc.; hydrogenated dimers of dienes [e.g. hydrogenated dimers of cycoalkadienes such as cyclopentadiene, cyclohexadiene, cycloheptadiene, etc. (e.g. perhydro-4,7-methanoindene etc.), butadiene dimer (vinylcyclohexene) and its hydrogenation product, and butadiene-cyclopentadiene dimer (vinylnorbornene) and its hydrogenation product]. The preferred bridged-ring hydrocarbon ring usually has a bornane, norbornane or adamantane skeleton.

The preferred Z ring is a bridged-ring hydrocarbon ring containing 2 to 4 component rings.

The Z ring (monocyclic or polycyclic hydrocarbon rings) usually have the substituent R⁴. The Z ring may have a plurality of substituents R⁴ and, in such cases, it is only necessary that at least one of R⁴s is any of the functional groups mentioned below, with the other R⁴ or R⁴s may each represent a hydrogen atom. Moreover, the R⁴ does not concurrently represent hydrogen atom and may be different according to n.

The substituent R⁴ is a hydrogen atom, a halogen atom (e.g. bromine, chlorine or fluorine), an alkyl group (e.g. C₁₋₄ alkyl groups such as methyl, ethyl, butyl, t-butyl, etc.), an oxygen-containing group, an amino group or an N-substituted amino group. The oxygen-containing group includes but is not limited to oxo group, hydroxy group, alkoxy groups (e.g. C₁₋₄ alkoxy groups such as methoxy, ethoxy, t-butoxy, etc.), carboxyl group, alkoxycarbonyl groups (e.g. C₁₋₄ alkoxycarbonyl groups such as methoxycarbonyl, ethoxycarbonyl, butoxycarbonyl, t-butoxycarbonyl, etc.), cycloalkyloxycarbonyl groups (e.g. C₃₋₁₀ cycloalkyloxycarbonyl groups such as cyclopropyloxycarbonyl, cyclobutyloxycarbonyl, cyclopentyloxycarbonyl, cyclohexyloxycarbonyl, cycloheptyloxycarbonyl, cyclooctyloxycarbonyl, etc.). aryloxycarbonyl groups (e.g.phenoxycarbonyl), aralkyloxycarbonyl groups (e.g. benzyloxycarbonyl, phenethyloxycarbonyl, trityloxycarbonyl, etc.), hydroxymethyl group, carbamoyl group, N-substituted carbamoyl groups (e.g. N-C₁₋₄ alkylcarbamoyl groups), and nitro group.

The preferred substituent R⁴ includes, for example, hydroxyl group, alkoxy groups, carboxyl group, alkoxycarbonyl groups and hydroxymethyl group. The still more preferred substituent R⁴ is hydroxyl group, carboxyl group or hydroxymethyl group (especially hydroxyl group or carboxyl group).

Those substituent groups R⁴ may each have been protected with a protective group, and this protective group may be a protective group which can be eliminated with an acid, that is to say a protective group which functions as a dissolution-inhibitory modifying group for preventing dissolution of the polymer prior to exposure to light.

The protective group for hydroxyl group and hydroxymethyl group includes but is not limited to alkoxycarbonyl groups (C₁₋₄ alkoxycarbonyl groups such as methoxycarbonyl, ethoxycarbonyl, t-butoxycarbonyl, etc.) and benzyloxy group.

The protective group for carboxyl group includes but is not limited to alkoxy groups (C₁₋₄ alkoxy groups such as methoxy, ethoxy, t-butoxy, etc.), aralkyloxy groups (benzyloxy, p-methoxybenzyloxy, diphenylmethyloxy, benzhydryloxy, etc.) and N-hydroxysuccinimido group.

When the substituent R⁴ is an oxygen-containing group,. it contributes to an improved adhesion between the resist and the substrate.

The symbol n represents an integer of not less than 1 (for example, 1 to 6), preferably 1 to 4 (e.g. 2 to 3), and more preferably 2 to 4.

In the above-mentioned compounds, the preferred substituents and combinations thereof are as follows.
(i)In at least one of the component rings forming the Z ring, the substituent R⁴ is an oxygen-containing group, for example at least one member selected from hydroxyl group, alkoxy groups, carboxyl group, alkoxycarbonyl groups and hydroxymethyl group.
(ii)In the formula (1), R¹ is a hydrogen atom or a straight-chain or branched-chain C₁₋₄ alkyl group (especially a hydrogen atom) and R² is a hydrogen atom or a straight-chain or branched-chain C₁₋₄ alkyl group.
(iii)In the formulas (1), (2), (1a), (2a), (11) and (12), R¹ is a 1-methyl-C₁₋₄ alkyl group.

Such acid-responsive compounds include (meth)acrylic esters of the following formulas. 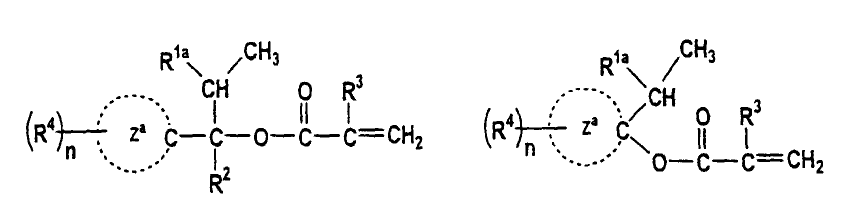 wherein R^{1a} represents a C₁₋₃ alkyl group; R³ represents a hydrogen atom or a methyl group; the Z^{a} ring represents a bridged alicyclic hydrocarbon ring having the substituent R⁴; R² is as defined hereinbefore.

When, in the formulas (1), (2), (1a), (2a), (11) and (12), the Z ring has no substituent R¹ is a branched-chain alkyl group having a tertiary carbon atom.

The acid-responsive (acid-sensitive) compounds (1) and (2) of the present invention can be prepared, for example, in accordance with the following reaction schemes. 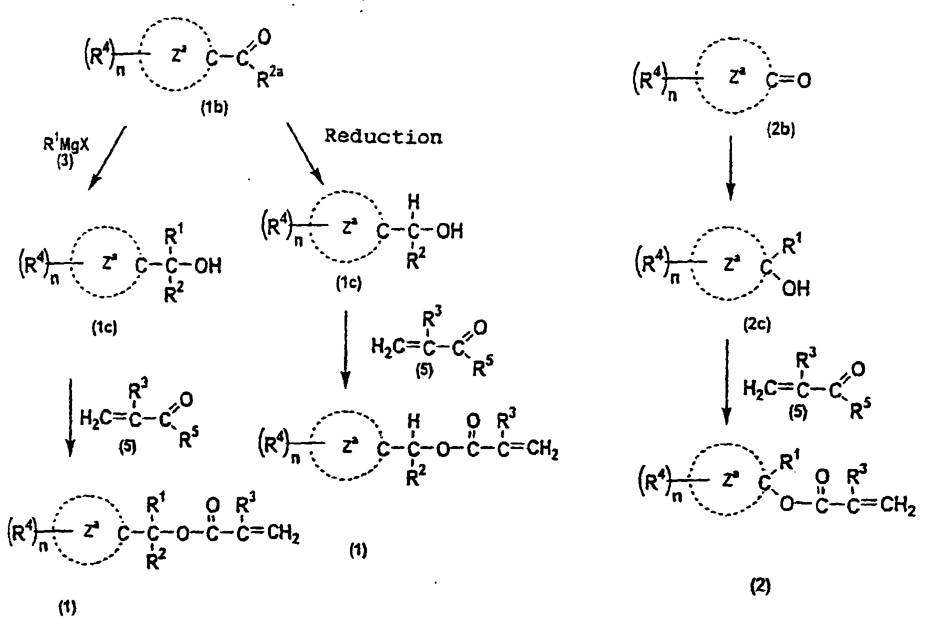 wherein X represents a halogen atom; R^{2a} means the same as R² or represents a halogen atom; R⁵ represents a halogen atom or a hydroxyl group, an alkoxy group, an alkenyloxy group or an alkynyloxy group; R¹, R², R³, R⁴ and the Z ring are each as defined hereinbefore.

The halogen atom typically includes chlorine, bromine and iodine, and the alkoxy group includes but is not limited to C₁₋₁₀, alkoxy groups (e.g. methoxy, ethoxy, t-butoxy, etc.). The alkenyloxy group includes but is not limited to C₂₋₁₀ alkenyloxy groups (e.g. vinyloxy, allyloxy, 1-propenyloxy, isopropenyloxy, 1-butenyloxy, 2-butenyloxy, 3-butenyloxy, 2-pentenyloxy, etc.), and the alkynyloxy group includes but is not limited to C₂₋₁₀ alkynyloxy groups (e.g. ethynyloxy, propynyloxy, etc.).

Referring to the above reaction schemes, the carbonyl compound (1b) includes, to mention typical examples, monocyclic compounds (cycloalkyl-1-C₂₋₆ alkanones such as cyclohexyl-1-ethanone etc.), spiro compounds (e.g. spiro[4.5]decan-8-yl-1-ethanone, spirobicyclohexan-9-yl-1-ethanone, etc.), ring assembly compounds (e.g. bicycloalkyl-1-C₂₋₆ alkanones such as bicyclohexan-4-yl-1-ethanone etc.), fused-ring compounds (e. g. perhydronaphthyl-1-ethanone, perhydrophenanthrenyl-1-ethanone, etc.), bridged-ring compounds (e.g. bicyclic compounds such as bornan-2-yl-1-ethanone, bornan-3-yl-1-ethanone, norbornan-2-yl-1-ethanone, etc. and tricyclic compounds such as adamantyl-1-C₂₋₆ alkanones, e.g. adamantan-1-yl-ethan-1-one, adamantan-1-yl-propan-1-one, adamantan-1-yl-butan-1-one, methyladamantan-1-yl-ethan-1-one, etc.); and derivatives from hydrogenated dimers of dienes (e.g. perhydro-4,7-methanoindenyl-1-C₂₋₆ alkanones such as perhydro-4,7-methanoinden-1-yl-1-ethanone etc).

The carbonyl compound (1b) in which R^{2a} is a halogen atom, i.e. the acid halide, includes but is not limited to monocyclic compounds (cyclohexanecarbonyl halides etc.), spiro compounds (spiro[4.5]decane-8-carbonyl halides etc.), ring assembly compounds (bicyclohexane-4-carbonyl halides etc.), fused-ring compounds (perhydronaphthalene-1-carbonyl halides, perhydrophenanthrene-1-carbonyl halides etc.), bridged-ring compounds (bicyclic compounds such as bornane-2-carbonyl halides, norbornane-2-carbonyl halides, etc. and tricyclic compounds such as adamantane-1-carbonyl halides etc. ), and derivatives from hydrogenated dimers of dienes (perhydro-4,7-methanoindene-1-carbonyl halides etc.).

The carbonyl compound (2b) includes but is not limited to monocyclic ketones (e.g. cycloalkanones such as cyclohexanone, methylcyclohexanone, etc.), spiro-ring ketones (spiro[4.5]decan-8-one, spirobicyclo-hexan-9-one, etc.), ring assembly ketones (e.g. bicycloalkanones such as bicyclohexan-4-one etc.), fused-ring compounds (perhydronaphthalen-1-one, perhydronaphthalen-2-one, perhydrophenanthren-1-one, etc.), bridged-ring compounds (e.g. bicyclic compounds such as bornan-2-one, bornan-3-one, norbornan-2-one and tricyclic compounds such as adamantanone, methyladamantanone, dimethyladamantanone, etc.), and derivatives from hydrogenated dimers of dienes (e.g. perhydro-4,7-methanoinden-1-one etc.).

In the above reaction schemes, the reaction of the carbonyl compound (1b) or (2b) with the reagent R¹MgX (3) can be carried out according to the conventional Grignard reaction. The amount of the Grignard reagent R¹MgX (3) relative to 1 mol of the carbonyl compound (1b) or (2b) may for example be about 0.8 to 3 mols (e.g. 1 to 2.5 mols), preferably about 1 to 2 mols, more preferably about 1 to 1.5 mols. Then the carbonyl compound (1b) to be used has a halogen atom for R^{2a}, the compound (1c) wherein R² is the same as R¹ can be produced by reacting 2 mols of the Grignard reagent R¹MgX (3) relative to 1 mol of the carbonyl compound (1b).

This reaction can be conducted in an inert solvent to the reaction, such as hydrocarbons (hexane, cyclohexane, etc.), ethers (dimethyl ether, diethyl ether, tetrahydrofuran, etc.), to mention just a few preferred solvents. The reaction temperature can be suitably selected from the range of, for example, about 0 to 100°C, preferably about 10 to 50°C.

The reduction reaction of carbonyl compound (1b) (wherein the carbonyl compound corresponding to R^{2a}=R²) with a reducing agent can be carried out according to a conventional method, for example by catalytic hydrogenation using hydrogen as the reducing agent or by the reduction reaction using a hydrogenation reducing agent.

The catalyst for the catalytic hydrogenation includes but is not limited to elemental metals (simple metals) such as platinum, palladium, nickel, cobalt, iron and copper and compounds containing such metal elements (e.g. platinum oxide, palladium black, palladium-carbon, copper chromite, etc.). The amount of the catalyst is generally about 0.02 to 1 mol relative to 1 mol of the substrate. The reaction temperature for catalytic hydrogenation may for example be about -20°C to 10°C (e.g. 0 to 70°C). The hydrogen pressure is generally 1 to 10 atmospheres in many instances.

Referring to the reduction reaction using a hydrogenation reducing agent, the hydrogenation reducing agent which can be used includes but is not limited to aluminum hydride, lithium aluminum hydride, sodium boron hydride, lithium boron hydride and diborane. The amount of the hydrogenation reducing agent used relative to 1 mol of the substrate is generally not less than 1 mol (for example, about 1 to 10 mols) in many instances. In many cases of reduction using a hydrogenation reducing agent, the reaction temperature is generally about 0 to 200°C (for example 0 to 170°C).

The above reduction reaction (catalytic hydrogenation method, the process using a hydrogenation reducing agent) may be carried out in a solvent inert to the reduction reaction (for example, hydrocarbons, carboxylic acids, ethers, esters and amides).

The hydroxy compound (1c) or (2c) formed by the reaction, optionally isolated, is subjected to the esterification reaction using (meth)acrylic acid or a derivative thereof (5) to give the acid-responsive compound (1) or (2).

The (meth)acrylic acid or its derivative (5), mentioned above, includes (meth)acrylic acid, (meth)acrylic anhydride, and reactive derivatives having a leaving group (e.g. acid halides ((meth)acryloyl chloride, (meth)acryloyl bromide, etc.), (meth)acrylic acid alkyl esters such as C₁₋₆ alkyl (meth)acrylates (e.g.methyl (meth)acrylate, ethyl (meth)acrylate, propyl(meth)acrylate, butyl(meth)acrylate, isobutyl (meth)acrylate, t-butyl (meth)acrylate, etc.), (meth)acrylic acid alkenyl esters (e.g. C₂₋₁₀ alkenyl (meth)acrylates such as vinyl (meth)acrylate, allyl (meth)acrylate, 1-propenyl(meth)acrylate, isopropenyl (meth)acrylate, 1-butenyl (meth)acrylate, 2-butenyl (meth) acrylate, 3-butenyl (meth)acrylate, 2-pentenyl (meth)acrylate, etc.), and alkynyl esters of (meth)acrylic acid (e.g. C₂₋₁₀ alkynyl (meth)acrylates such as ethynyl (meth)acrylate, propynyl (meth)acrylate, etc. )].

The preferred compound (5) includes (meth)acrylic acid, (meth)acryloyl halides, C₁₋₆ lower alkyl esters of (meth)acrylic acid, C₂₋₆ alkenyl esters of (meth)acrylic acid, and C₂₋₆ alkynyl esters of (meth)acrylic acid. Particularly with a (meth)acryloyl halide or a C₂₋₆ alkenyl (meth)acrylate, the corresponding acid-responsive compound can be obtained with high selectivity and in high yield through a leaving group exchange reaction while side reactions such as addition polymerization are inhibited.

The above esterification reaction can be carried out by a conventional manner, for example in the presence of a suitable catalyst (an acid catalyst). When a (meth)acryloyl halide is used, there are cases in which the acid-responsive compound is contaminated with the halogen component. Therefore, this esterification reaction is preferably effected by the esterification reaction using (meth)acrylic acid or the transesterification reaction. The esterification reaction and transesterification reaction can be conducted using the conventional esterification catalyst (for example a non-halogen series inorganic acid such as sulfuric acid, hydrochloric acid, a sulfonic acid such as p-toluenesulfonic acid, a protonic acid such as acidic ion exchange resin, a Lewis acid such as boron trifluoride, an enzyme, etc.) and a transesterification catalyst (for example, the esterification catalysts, alkali metal alkoxide such as sodium alkoxides, aluminum alkoxides, titanic acid esters, etc.).

To enhance the reaction efficiency and obtain the objective acid-responsive compound in high yield, the esterification reaction (inclusive of leaving group exchange reactions such as transesterification) between the hydroxy compound (1c) or (2c) and (meth)acrylic acid or a derivative thereof (5) is conducted with advantage in the presence of a catalyst comprised of a compound of a Group 3 element of Periodic Table of the Elements. In the reaction utilizing such a catalyst,the formation of the amine hydrochloride can be inhibited and, when a C₁₋₄ lower alkyl ester or C₂₋₄ alkenyl ester of (meth)acrylic acid is used, the objective compound can be protected against contamination with the halogen component. Moreover, because a low-boiling compound (e.g. the above ester) can be used as the (meth)acrylic acid or derivative (5),the treatment after the reaction is easy and the isolation yield can be dramatically increased.

Referring to the catalyst comprised of a compound of Group 3 element, the Group 3 element includes rare earth elements, e.g. scandium, yttrium, lanthanide series elements (lanthanum, cerium, praseodymium, neodymium, promethium, samarium, europium, gadolinium, terbium, dysprosium, holmium, erbium, thulium, ytterbium, lutetium)], and actinoid series elements (e.g. actinium etc.). The preferred Group 3 element includes rare earth elements, such as scandium, yttrium, and lanthanoid series elements (samarium, gadolinium, ytterbium, etc.).

The valence of the Group 3 element is not particularly restricted but is often bivalent to tetravalent, particularly bivalent or trivalent. The compound of Group 3 element, mentioned above, is not particularly restricted only if it has the necessary catalyst activity, and may be an elemental metal or a compound or complex of the metal with an inorganic compound (e.g. a halide, oxide, double oxide, phosphorus compound or nitrogen compound) or an organic compound (e.g. an organic acid). In many instances, it is the hydroxide or oxo-acid salt, organic acid salt, inorganic acid salt or halide containing the metal element or a coordination compound (complex) containing the metal element. The complex may be a π complex such as a metallocene compound. Furthermore, the compound of Group 3 element may be a double salt compound (complex metal compound) with another metal. Those catalysts can be used each alone or in a combination of two or more species.

The catalyst component is now described in further detail taking a samarium compound as an example, it being, however, to be understood that the compounds of other Group 3 elements which correspond to the samarium compound can be used likewise with success.

The hydroxide includes samarium (II) hydroxide and samarium (III) hydroxide, for instance, and the metal oxide includes samarium (II) oxide and samarium (III) oxide, for instance.

The organic acid salt includes but is not limited to salts with such organic acids as organic carboxylic acids (monocarboxylic acids, polycarboxylic acids), hydroxycarboxylic acids, thiocyanic acid or sulfonic acids (alkylsufonic acids, benzenesufonic acid, arylsulfonic acids, etc.). The inorganic acid salt includes but is not limited to the nitrate, sulfate, phosphate, carbonate and perchlorate. The organic acid salt and inorganic acid salt, mentioned above, include but are not limited to samarium acetate, samarium trichloroacetate, samarium trifluoroacetate, samarium trifluoromethanesulfonate (i.e. samarium triflate), samarium nitrate, samarium sulfate, samarium phosphate and samarium carbonate.

The halide may for example be the fluoride, chloride, bromide or iodide.

The ligand forming the complex includes OH (hydroxo), alkoxy, acyl, alkoxycarbonyl, acetylacetonato, cyclopentadienyl, C₁₋₄ alkyl-substituted cyclopentadienyl (e.g. C₁₋₂ alkyl-substituted cyclopentadienyl groups such as pentamethylcyclopentadienyl etc.), dicyclopentadienyl, C₁₋₄ alkyl-substituted dicyclopentadienyl (e.g. C₁₋₂ alkyl-substituted dicyclopentadienyl such as pentamethyldicyclopentadienyl etc.), halogen, CO, CN, oxygen, H₂O (aqua), phosphorus compounds such as phosphines, nitrogen-containing compounds such as NH₃ (ammine), NO, NO₂ (nitro), NO₃ (nitrato), ethylenediamine, diethylenetriamine, pyridine, phenanthroline, etc. Referring to the complexes or complex salts, one or more similar or dissimilar ligands may be coordinated.

Among the complexes, samarocene complexes include diacetylacetonatosamarium (II), triacetylacetonatosamarium (III), dicyclopentadienylsamarium (II), tricyclopentadienylsamarium (III), dipentamethylcyclopentadienylsamarium (II) and tripentamethylcyclopentadienylsamarium (III).

When the compound of Group 3 element [the bivalent samarocene complex having the pentamethylcyclopentadienyl ligands which are highly electron-donative [(C₅Me₅)₂Sm;(PMSm)] or any of samarium compounds such as samarium halides, alkoxide, hydroxide, etc.] is used as the catalyst, esterification proceeds with a higher efficiency and with side reactions inhibited as compared with the reaction in the presence of a Lewis acid catalyst or a protonic acid catalyst even in esterification reactions which are handicapped as equilibrium reactions. Thus, this catalyst is useful for producing the acid-responsive compounds (1) and (2) by a leaving group exchange reaction such as transesterification.

The catalyst comprised of a compound of Group 3 element may be a homogeneous system or a heterogeneous system. Moreover, the catalyst may be an solid catalyst comprising the compound of Group 3 element supported on a support or carrier. The support is usually a porous support such as activated carbon, zeolite, silica, silica-alumina, bentonite or the like. The amount of the supported catalyst component is about 0.1 to 50 parts by weight, preferably about 0.5 to 30 parts by weight, more preferably about 1 to 20 parts by weight relative to 100 parts by weight of the support.

The amount of the catalyst (e.g. the catalyst comprised of the compound of Group 3 element) can be liberally selected from a broad range, for example the range of about 0.1 mol % to 1 equivalent, preferably about 0.5 to 50 mol %, more preferably about 1 to 25 mol % (e.g. about 5 to 20 mol %), based on the hydroxy compound (1c) or (2c).

The esterification reaction mentioned above (particularly the reaction using the compound of Group 3 element as the catalyst) may be conducted in the presence of an oxime. The oxime may be whichever of an aldoxime or a ketoxime and includes aliphatic oximes such as 2-hexanone oxime etc., alicyclic oximes such as cyclohexanone oxime etc., and aromatic oximes such as acetophenone oxime, benzophenone oxime, benzyl dioxime, etc.

The amount of the oxime can be selected from a broad range, for example the range of about 0.1 mol % to 1 equivalent, preferably about 1 to 50 mol %, more preferably about 5 to 40 mol % (e.g. about 5 to 30 mol %), based on the hydroxy compound (1c) or (2c).

The ratio of (meth)acrylic acid or its derivative (5) to hydroxy compound (1c) or (2c) is about 0.5 to 5 mols, preferably about 0.8 to 5 mols, more preferably not less than 1 mol (e.g. about 1 to 3 mols, particularly about 1 to 1.5 mols) of (meth)acrylic acid or derivative (5) per equivalent of hydroxy compound (1c) or (2c) (that is the weight of the hydroxy compound per hydroxyl group). Since the esterification reaction is an equilibrium-controlled reaction, it is more advantageous to use a larger proportion of (meth)acrylic acid or derivative thereof (5) for accelerating the reaction but because of the usually high catalyst activity of the compound of Group 3 element. (meth)acrylic acid or derivative thereof (5) need not be used in a large excess. Particularly, in the reaction involving a combination of reactants which is very unfavorable from the standpoint of reaction equilibrium, the use of the alkenyl ester having a vinyl leaving group (e.g. vinyl ester) as the (meth)acrylic acid or derivative thereof (5) rather leads, in many instances, to an early completion of reaction and a better result even if the compound (1c) is used in a proportion of only 1 mol or less (e.g. 0.4 to 1 mol, particularly 0.5 to 1 mol) per equivalent of the leaving group of hydroxy compound (1c) or (2c).

In the process using the catalyst, the heat of reaction is not so high as in the process using an acid halide such as (meth)acryloyl chloride so that the reaction can be smoothly conduced in a small amount of solvent and the end product can be obtained in a higher yield.

The esterification reaction mentioned above can be carried out in the presence or absence of a solvent inert to the reaction. The reaction solvent which can be used includes but is not limited to aliphatic hydrocarbons, alicyclic hydrocarbons, aromatic hydrocarbons, polar aprotic solvents such as ketones, ethers, amides, N-methylpyrrolidone, nitriles, etc. and mixed solvents thereof. As the reaction solvent, (meth)acrylic acid or derivative thereof (5) may be used.

When a highly hydrophilic species of hydroxy compound (1c) or (2c) is used, the solvent may be a hydrophilic solvent (ketones such as acetone, methyl ethyl ketone, etc.. ethers such as dioxane, diethyl ether , tetrahydrofuran, etc., and other polar aprotic solvents), or a mixed solvent of a hydrophilic solvent and a hydrophobic solvent (an aliphatic, alicyclic or aromatic hydrocarbon).

Since the above reaction is an equilibrium-controlled reaction, it is advantageous to remove the reaction-interfering components such as the cleaved (eliminated) component from the reaction system for accelerating the reaction. For removal of the eliminated component, it is advantageous to use a high-boiling solvent (for example an organic solvent boiling at 50 to 120°C, particularly about 60 to 115°C) or an azeotropic solvent (for example the hydrocarbons).

The esterification reaction temperature can be selected from the range of, for example, about 0 to 150°C, preferably about 25 to 120°C. When the catalyst comprised of the compound of Group 3 element is used, the acid-responsive compound forms with high efficiency even under mild conditions. Thus, the reaction temperature may for example be about 0 to 150°C, preferably about 10 to 100°C, more preferably about 20 to 80°C. Particularly when the alkenyl ester, for instance, is used as the (meth)acrylic acid or derivative (5), the reaction can be smoothly carried through even under mild conditions, namely at about 20 to 50°C. The reaction can be conduced at atmospheric pressure, under applied pressure or at elevated pressure. Moreover, the reaction can be carried out batchwise, semi-batchwise or continuously in the conventional manner.

After completion of the reaction, the acid-responsive compound (1) or (2) can be easily isolated and purified by such separatory means as filtration, concentration, distillation, extraction, crystallization, recrystallization, column chromatography, etc. as applied independently or in combination.

### [Photoresist resin composition]

The photoresist resin composition of this invention is characterized in that at least a polymer containing the unit of formula (11) or (12) (e.g. a unit having an adamantane skeleton) and a photoactive acid precursor (photoactive acid generator) are used in combination and that the polymer is solubilized by irradiation with light. Thus, probably because the ester bond (linkage) which is adjacent to the bulky, hydrophobic Z ring is stably eliminated with high efficiency by the acid produced by the irradiation, a fine-line resist pattern can be formed with high accuracy and reproducibility while the high sensitivity and high etching resistance are maintained.

Furthermore, because a polymer containing the acid-responsive compound (1) or (2) as a repeating unit is highly soluble in a organic solvent for photoresist use, the precipitation of the polymer can be prevented and the stability of the photoresist solution be improved. In addition, because of the high adhesion of the solution to the substrate, resist patterns can be obtained with high accuracy. Moreover, the cleaning and removing by a development procedure such as dry etching after pattern exposure can be neat and thorough so that the sensitivity of pattern formation is high.

Particularly when the substituent group R⁴ on the Z ring has a hydroxyl, alkoxy, carboxyl, alkoxycarbonyl or hydroxymethyl group or a group derived from any of such groups, marked improvements can be realized in the above-mentioned characteristics. Moreover, when the Z ring is an alicyclic hydrocarbon ring, the dry-etching resistance is high and particularly when it is a polycyclic hydrocarbon ring, the degree of developer-induced swelling is low so that the circuit pattern can be formed with good accuracy.

The polymer mentioned above may be a homopolymer or a copolymer of the acid-responsive compound or compounds represented by formula (1) or/and (2) or a copolymer of the acid-responsive compound or compounds of formula (1) or/and (2) with one or more other copolymerizable monomers.

The copolymerizable monomers mentioned above include but are not limited to (meth)acrylic monomers (e.g.(meth)acrylic C₁₋₁₀ alkyl esters such as methyl (meth)acrylate, ethyl (meth)acrylate, isopropyl (meth)acrylate, butyl (meth)acrylate, s-butyl (meth)acrylate,t-butyl (meth)acrylate, hexyl(meth)acrylate, octyl(meth)acrylate, 2-ethylhexyl (meth)acrylate, etc.; (meth)acrylic hydroxy C₂₋₆ alkyl esters such as 2-hydroxyethyl (meth)acrylate, 2-hydroxypropyl (meth)acrylate, etc.; glycidyl (meth)acrylate, (meth)acrylonitrile, cyclohexyl (meth) acrylate, phenyl (meth)acrylate, etc.), styrenic monomers (styrene, α-methylstyrene, vinyltoluene, etc.), vinyl ester monomers (vinyl acetate, vinyl propionate, etc.), carboxyl group-containing monomers ((meth)acrylic acid, maleic anhydride, itaconic acid, maleic monoesters, etc.), sulfonic acid group-containing monomers (styrenesulfonic acid etc.), lactone ring-containing monomers, and monomers having an alicyclic hydrocarbon ring.

The lactone ring-containing monomers include but are not limited to (meth)acrylic monomers of the following formula: 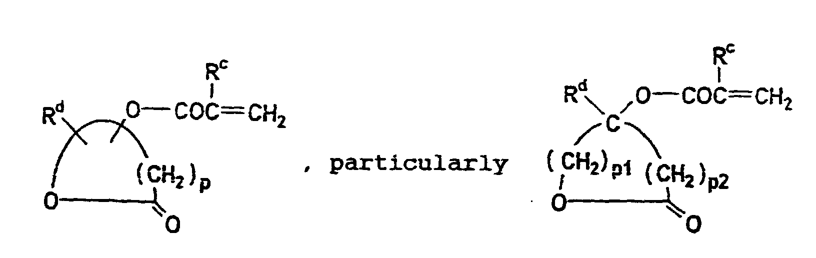 wherein R^{c} represents a hydrogen atom or a methyl group; R^{d} represents a hydrogen atom or a C₁₋₄ alkyl group; p represents an integer of about 2 to 15; p1 and p2 each represents an integer of about 0 to 8; p1+p2 = about 1 to 14
and the allyl monomers corresponding to the above monomers.

The C₁₋₄ alkyl group mentioned for R^{d} includes methyl, ethyl, propyl, isopropyl, butyl, isobutyl and t-butyl. R^{d} is usually a hydrogen atom or a methyl group. The integer p is usually about 3 to 10, particularly about 3 to 6. Moreover, p1 and p2 each is usually an integer of 0 to 8, and p1+p2 = about 2 to 9 (preferably 2 to 5). The numbers and substitution positions of the (meth)acryloyloxy group, allyloxy group and Rd are not particularly restricted; thus, they may be located in suitable positions on the lactone ring.

The monomer having an alicyclic hydrocarbon ring includes but is not limited to (meth)acrylates having a monocyclic hydrocarbon ring and (meth)acrylates having a polycyclic hydrocarbon ring (spiro hydrocarbon ring, ring assembly hydrocarbon ring, fused-ring hydrocarbon ring and bridged-ring hydrocarbon ring). The (meth)acrylates having a monocyclic hydrocarbon ring include but are not limited to C₄₋₁₀ cycloalkyl (meth)acrylates such as cycloheptyl (meth)acrylate, cyclohexyl (meth)acrylate, cyclopentyl (meth)acrylate, and cyclooctyl (meth)acrylate. The (meth)acrylates having a spiro hydrocarbon ring include but are not limited to (meth)acrylates having a spiro C₈₋₁₆ hydrocarbon ring, such as spiro[4.4]nonyl (meth)acrylate, spiro[4.5]decanyl (meth)acrylate and spirobicyclohexyl (meth)acrylate. The (meth)acrylates having a ring assembly hydrocarbon ring include but are not limited to (meth)acrylates having C₅₋₁₂ cycloalkane rings such as bicyclohexyl (meth)acrylate, and the (meth)acrylates having a fused-ring hydrocarbon ring include but are not limited to (meth)acrylates having a 5 to 8-membered cycloalkane fused ring, such as perhydronaphthyl (meth)acrylate, perhydroanthryl (meth)acrylate, etc.

The (meth)acrylates having a bridged-ring hydrocarbon ring include but are not limited to (meth)acrylates having a bicyclic hydrocarbon ring, such as bornyl (meth)acrylate, norbornyl (meth)acrylate, isobornyl (meth)acrylate, isobornyloxyethyl (meth)acrylate, etc.; (meth)acrylates having a tricyclic hydrocarbon ring, such as dicyclopentadienyl (meth)acrylate, dicyclopentenyl (meth)acrylate, dicyclopentenyloxyalkyl (meth)acrylates, tricyclodecanyl (meth)acrylate, tricyclo [5.2.1.0^{2,6}] decanyl (meth)acrylate, tricyclodecanyloxyethyl (meth)acrylate, tricyclo [4.3.1.1^{2,5}] undecanyl (meth)acrylate, adamantyl (meth)acrylate, etc.; and (meth)acrylates having a tetracyclic hydrocarbon ring such as a tetracyclo[4.4.0.1^{2,5}.1^{7,10}]dodecane ring, a perhydro-1,4-methano-5,8-methanonaphthalene ring, etc.

Those copolymerizable monomers may have a variety of substituents (e.g. polar groups such as oxo, hydroxyl, alkoxy, carboxyl, alkoxycarbonyl, cycloalkyloxycarbonyl, aryloxycarbonyl, aralkyloxycarbonyl, hydroxymethyl, carbamoyl, N-substituted carbamoyl, nitro, etc.). Those copolymerizable monomers can be used each alone or in a combination of two or more species.

The proportion of the acid-responsive compound (1), (2) in the copolymer may for example be about 10 to 100 weight % (e.g. 15 to 90 weight %), preferably about 25 to 100 weight % (e.g. 30 to 75 weight %), more preferably about 30 to 100 weight % (e.g. 35 to 70 weight %).

The photoactive acid precursor which can be used includes the conventional compounds which, when exposed to light, form acids (protonic acids or Lewis acids) with high efficiency, for example diazonium salts, iodonium salts, sulfonium salts, oxathiazole derivatives, s-triazine derivatives, imide compounds, oxime sulfonates, diazonaphthoquinone, sulfonic esters [1-phenyl-1-(4-methylphenyl)sulfonyloxy-1-benzoylmethane, 1,2,3-trisulfonyloxymethylbenzene, 1,3-dinitro-2-(4-phenylsulfonyloxymethyl)benzene, 1-phenyl-1-(4-methylphenyl)sulfonyloxymethyl-1-hydroxy-1-benzoylmethane, disulfone derivatives (diphenyl disulfone etc.), benzoin tosylate, etc.] or Lewis acids (triphenylsulfonium hexafluoroantimonate ((Ph)₃S⁺SbF₆⁻), triphenylsulfonium hexafluorophosphate ((Ph)₃S⁺PF₆⁻), triphenylsulfonium methanesulfonyl ((Ph)₃S⁺CH₃SO₃⁻), diphenyliodonium hexafluorophosphate, etc.). In the above description, Ph represents a phenyl group.

Those photoactive acid precursors can be used each alone or in a combination of two or more species.

The amount of the photoactive acid precursor can be selected according to the strength of the acid that will be produced by irradiation and the amount of the acid-responsive compound, among other conditions, for example from the range of about 0.1 to 30 parts by weight, preferably about 1 to 25 parts by weight, more preferably about 2 to 20 parts by weight, relative to 100 parts by weight of the polymer.

The photoresist resin composition may contain an alkali-soluble component such as alkali-soluble resin (novolac resin, phenolic resin, carboxyl-containing resin, etc.), a coloring agent (dye), an organic solvent, etc. The organic solvent includes but is not limited to hydrocarbons, halogenated hydrocarbons, alcohols, esters, ketones, ethers, cellosolves (methylcellosolve, ethylcellosolve, butylcellosolve, etc.), carbitols, glycol ether esters (mono-or polyalkylene glycol monoalkyl ether esters, cellosolve acetates such as ethylcellosolve acetate, propylene glycol monomethyl ether acetate, propylene glycol monoethyl ether acetate, etc.) and mixed solvents thereof. The polymer containing the acid-responsive compound (the compound in which the Z ring has the substituent R⁴) as a constituent unit is characteristically well-soluble in the organic solvent component of the photoresist.

Further, the photoresist resin composition may be freed of contaminants by the cobventional separation and purification procedure such as the use of a filter.

The photoresist resin composition of this invention can be prepared by mixing the polymer and the photoactive acid precursor. As this photoresist resin composition is coated on a substrate or base and dried and the resulting layer (resist layer or resist film) on the substrate is imagewise exposed to light and developed, a pattern corresponding to the exposure pattern can be formed on the substrate, with high sensitivity to exposure light and high pattern resolution. Usually, the film is exposed to light through a suitable mask to form a latent image, which can then be developed to provide a fine-line pattern with high accuracy.

The substrate or base can be selected according to the intended use of the photoresist resin composition and may for example be a silicon wafer or a metal, plastic, glass, ceramic or other substrate. The coating with the photoresist resin composition can be effected by the conventional procedure suited to each application, for example by the spin coating technique or the roll coating technique. The thickness of coating layer comprising the photoresist resin composition can be judiciously selected from the range of, for example, about 0.1 to 20 µm.

For the exposure to light, light beams of various wavelengths, e.g. ultraviolet rays and X-rays, can be employed. For the exposure of resists for semiconductor manufacturing, g-line, i-line, and excimer laser (e.g. XeCl, KrF, KrCl, ArF, Arcl, etc.) beams can be utilized.

The exposure beam energy can be selected from the range of, for example, about 1 to 1000 mJ/cm², preferably about 10 to 500 mJ/cm².

On irradiation, an acid is generated from the acid precursor and the group containing the Z ring (usually the alcohol containing the Z ring) is eliminated by the generated acid and a carboxyl group contributory to solubilization is generated. Therefore, the desired pattern can be formed using an aqueous developer or an alkali developer. Particularly because the photoresist resin composition of the present invention has the Z ring, such as an adamantane skeleton, it is highly resistant to etching (dry etching in particular) so that an elaborate circuit pattern can be formed with high accuracy. Incidentally, the cleavage of the group containing the Z ring may be promoted by exposure and post-exposure baking (PEB).

The present invention can be embodied in various applications, such as circuit-forming materials (resists for semiconductor manufacturing, printed circuit boards, etc.) and image-forming materials (printing plates, relief images, etc.).

### [Introduction of functional groups]

The acid-responsive compounds (1) and (2) and the polymer component units (11) and (12) have the substituent R⁴ as mentioned above. The substituent R⁴ can be introduced in a suitable stage among the above-described reaction stages or after completion of the relevant reaction. The hydroxyl group, for instance, can be given by a conventional oxidation process using nitric acid or chromic acid, an oxidation process using oxygen with a cobalt salt as the catalyst or a biochemical oxidation process, for instance, or generated by a method which comprises introducing a halogen atom (e.g. a bromine atom) and then carrying out a hydrolysis reaction using an inorganic salt such as silver nitrate or silver sulfate to thereby introduce a hydroxyl group. As a preferred method, the method using an oxidation catalyst as described in Japanese Patent Application Laid-Open No.327626/1997, for instance, can be mentioned. In this oxidation method, the substrate compound of formula (1a), (2a), (1b), (2b), (1c) or (2c) or the polymer component unit (11) or (12) can be oxidized with oxygen in the presence of an oxidation catalyst comprised of a certain imide compound or an oxidation catalyst comprised of the imide compound and a co-catalyst to thereby introduce a hydroxyl group.

The imide compound mentioned above includes compounds having N-hydroxyimide groups (e.g. aliphatic, alicyclic and aromatic compounds each having about 1 to 3 N-hydroxyimido groups), such as N-hydroxysuccinimide, N-hydroxymaleimide, N-hydroxyhexahydrophthalimide, N,N'-dihydroxycyclohexanetetracarboximide, N-hydroxyphthalimide, N-hydroxytetrabromophthalimide, N-hydroxytetrachlorophthalimide, N-hydroxyhetimide, N-hydroxyhimimide, N-hydroxytrimellitimide, N,N'-dihydroxypyromellitimide, and N,N'-dihydroxynaphthalenetetracarboximide. Among the preferred compounds are N-hydroxyimide compounds derived from alicyclic polycarboxylic anhydrides, particularly those derived from aromatic polycarboxylic anhydrides, for example N-hydroxyphthalimide.

Such imide compounds have high oxidizing activity and are capable of promoting an oxidation reaction catalytically even under mild condition. Furthermore, by oxidizing a substrate in the presence of the imide compound and a co-catalyst, a hydroxyl group can be introduced with high efficiency.

The co-catalyst includes metal compounds, for example compounds containing elements of Group 2A of Periodic Table of the Elements, transition metal elements (e.g. Group 3A elements, Group 4A elements, Group 5A elements, Group 6A elements, Group 7A elements, Group 8 elements, Group 1B elements, and Group 2B elements) and elements of Group 3B (boron B, aluminum Al, etc.) of the Periodic Table of Elements.

The preferred co-catalyst includes Group 4A elements such as Ti, Zr, etc., Group 5A elements such as V, Group 6A elements such as Cr, Mo, W, etc., Group 7A elements such as Mn, Tc, Re, etc., Group 8 elements such as Fe, Ru, Co, Rh, Ni, etc., and Group 1B elements such as Cu.

The co-catalyst may for example be an elemental metal or a hydroxide thereof but, in many instances, metal oxides (double oxides or oxo-acid salts), organic acid salts, inorganic acid salts and halides containing the elements, coordination compounds (complexes) and polyacids (heteropolyacids and isopolyacids) or their salts containing the metal elements are used.

The oxidation catalyst comprised of the imide compound or the oxidation catalyst comprising the imide compound and co-catalyst may be a homogeneous system or a heterogeneous system. Moreover, the oxidation catalyst or oxidation catalyst system may be a solid catalyst comprising a catalytic component supported on a support or carrier.

The ratio of the co-catalyst to the imide compound can be selected from the range of, for example, about 0.001 to 10 mols of the co-catalyst relative to 1 mol of the imide compound. In order to maintain the activity level of the oxidation catalyst system high, the amount of the co-catalyst is preferably from an effective amount to 0.1 mol (for example. about 0.001 to 0.1 mol, preferably about 0.005 to 0.08 mol, more preferably about 0.01 to 0.07 mol) relative to 1 mol of the imide compound.

The amount of the imide compound relative to 1 mol of the substrate is about 0.001 to 1 mol (0.01 to 100 mol %), preferably about 0.001 to 0.5 mol (0.1 to 50 mol %), more preferably about 0.01 to 0.30 mol.

The amount of the co-catalyst relative to 1 mol of the substrate is about 0.0001 mol (0.1 mol %) to 0.5 mol, preferably about 0.001 to 0.3 mol, more preferably about 0.0005 to 0.1 mol (e.g. 0.005 to 0.1 mol), in many instances.

The oxygen for use in the oxidation reaction may be active oxygen but it is economically advantageous to use molecular oxygen. The molecular oxygen is not particularly restricted but may be pure oxygen or oxygen diluted with an inert gas such as nitrogen, helium, argon or carbon dioxide. Not only from the standpoint of workability (handling easiness)and safety but also from economic points of view, the use of air is preferable.

The amount of oxygen relative to 1 mol of the substrate is usually not less than about 0.5 mol (e.g. 1 mol or more), preferably about 1 to 100 mols, more preferably about 2 to 50 mols. Oxygen is often used in excess mol relative to the substrate and it is particularly advantageous to carry out the reaction in an atmosphere containing molecular oxygen such as air or oxygen gas.

The oxidation reaction is usually carried out in an organic solvent inert to the reaction. The organic solvent that can be used includes but is not limited to organic carboxylic acids such as acetic acid and hydroxycarboxylic acids, nitriles such as acetonitrile, benzonitrile, etc.,amides such as formamide, acetamide, dimethylformamide (DMF), dimethylacetamide, etc., alcohols such as t-butanol, t-amyl alcohol, etc., aliphatic hydrocarbons such as hexane, octane, etc., aromatic hydrocarbons such as benzene etc., halogenated hydrocarbons, nitro compounds, esters such as ethyl acetate etc., ethers such as diethyl ether, diisopropyl ether, dioxane, etc., and mixed solvents thereof.

When the oxidation reaction is carried out in the presence of a protonic acid, the reaction proceeds smoothly and the objective compound can be obtained with high selectivity and in good yield. The protonic acid may be utilized as the solvent as mentioned above. The protonic acid includes organic acids (e.g. organic carboxylic acids such as formic acid, acetic acid, propionic acid, etc., oxalic acid, hydroxycarboxylic acids such as citric acid, tartaric acid, etc., alkylsulfonic acids such as methanesulfonic acid, ethanesulfonic acid, etc., arylsulfonic acids such as benzenesulfonic acid, p-toluenesulfonic acid, etc.) and inorganic acids (e.g. hydrochloric acid, sulfuric acid, nitric acid, phosphoric acid, etc.).

The oxidation method using the oxidation catalyst or oxidation catalyst system is characterized in that the oxidation reaction proceeds smoothly even under comparatively mild conditions. The reaction temperature may for example be about 0 to 200°C, preferably about 30 to 150°C, and the reaction is generally conducted at about 50 to 120°C in many instances. This reaction can be conducted at atmospheric or under applied pressure.

When the above oxidation reaction is carried out in the presence of a strong acid, an oxo group can be introduced with high efficiency. The strong acid includes not only sulfuric acid and sulfonic acids mentioned above but also superacids, among others.

The introduction of a carboxyl group can be achieved by various reactions but it is advantageous to employ a technique (carboxylation process) similar to the above-described oxidation reaction process using the imide compound (or the imide compound and co-catalyst) as a catalyst except that carbon monoxide and oxygen are used in lieu of oxygen. The carbon monoxide and oxygen may be pure carbon monoxide and oxygen or carbon monoxide and oxygen as diluted with an inert gas beforehand in the same manner as mentioned for the oxidation reaction. As the oxygen source, air may be likewise employed.

The proportion of carbon monoxide can be selected from the range of not less than 1 mol (e.g. 1 to 1000 mols) relative to 1 mol of the substrate and is preferably an excess mole, for example about 1.5 to 100 mols (e.g. 2 to 50 mols), more preferably about 2 to 30 mols (e.g. 5 to 25 mols).

The proportion of oxygen relative to 1 mol of the substrate can be selected from the range of not less than 0.5 mol (e.g. 0.5 to 100 mols), preferably about 0.5 to 30 mols, more preferably about 0.5 to 25 mols.

It is preferable to use a larger amount of carbon monoxide than oxygen. The usual ratio of CO to O₂ is CO/O₂ = about 1/99 to 99/1 (mol %) (e.g. 10/90 to 99/1 (mol %)], preferably about 30/70 to 98/2 (mol%), more preferably about 50/50 to 95/5 (mol%), particularly about 60/40 to 90/10 (mol %).

A hydroxymethyl group can be generated by reducing the carboxyl group-introduced substrate with hydrogen or a hydrogenation reducing agent (e.g. sodium boron hydride-Lewis acid, aluminum hydride, lithium aluminum hydride, lithium trialkoxyaluminum hydrides, diborane, etc.).

A nitro group can be introduced in the conventionl manner, for example by a process using a nitrating agent (e.g. mixed acid of sulfuric acid and nitric acid; nitric acid; nitric acid and an organic acid (e.g. a carboxylic acid such as acetic acid); a nitrate and sulfuric acid; dinitrogen pentoxide, etc.).

The preferred nitration process may for example be a nitration process which comprises contacting the substrate with a nitrogen oxide in the presence or absence of the imide compound (or the imide compound and co-catalyst).

The nitrogen oxide mentioned above can be expressed by the formula NₓO_{y} (wherein x represents an integer of 1 or 2; y represents an integer of 1 to 6).

In the compound of the above formula, when x is equal to 1, y is usually an integer of 1 to 3 and when x is equal to 2, y is usually an integer of 1 to 6.

The nitrogen oxide, thus, includes but is not limited to N₂O, NO, N₂O₃, NO₂, N₂O₄, N₂O₅, NO₃ and N₂O₆. Those nitrogen oxides can be used each independently or in a combination of two or more species.

The preferred nitrogen oxide includes (1) the nitrogen oxide (particularly N₂O₃) produced by reacting at least one nitrogen compound selected from the group consisting of dinitrogen oxide (N₂O) and nitrogen monoxide (NO) with oxygen or a nitrogen oxide containing N₂O₃ as a main component and (2) nitrogen dioxide (NO₂) or a nitrogen oxide containing NO₂ as a main component.

The nitrogen oxide N₂O₃ can be easily obtained by reacting N₂O and/or NO with oxygen. More specificantly, it can be prepared by a process which comprises charging a reactor with nitrogen monoxide and oxygen to give N₂O₃ as a blue-colored liquid. Therefore, instead of preparing N₂O₃ in advance, the nitration reaction may be carried out by introducing N₂O and/or NO and oxygen into the reaction system.

Here, the oxygen may be either pure oxygen or oxygen diluted with an inert gas (carbon dioxide, nitrogen, helium, argon, etc.) beforehand. The oxygen source may be air.

In the reaction mode using nitrogen dioxide (NO₂), among the nitrogen oxides, the nitration reaction proceeds smoothly even without coexistence of oxygen. Therefore, the reaction system using NO₂ does not essentially require oxygen, although NO₂ may be used in combination with oxygen.

In the above oxidation process using the imide compound (or the imide compound and co-catalyst) as a catalyst, it is advantageous to carry out nitration using a nitrogen oxide (or a nitrogen oxide and oxygen) in lieu of oxygen in otherwise the same manner as the oxidation process described above.

The amount of the nitrogen oxide an be selected according to the amount of the nitro group to be introduced, for example from the range of about 1 to 50 mols, preferably about 1.5 to 30 mols, relative to 1 mol of the substrate, and is usually about 2 to 25 mols.

When the catalyst comprised of the imide compound is used. the nitration reaction proceeds smoothly even under comparatively mild conditions. The reaction temperature can be selected according to the kinds of imide compound and substrate, for example from the range of about 0 to 150°C, preferably about 25 to 125°C, more preferably about 30 to 100°C. The nitration reaction can be carried out at atmospheric pressure or under applied pressure.

The nitro-group introduced into the substrate can be converted to an amino group by reduction. This reduction reaction can be carried out in the conventional manner, for example in the same manner as the reduction reaction for converting the carbonyl compound (1b) to the hydroxy compound (1c) according to the reaction scheme presented hereinbefore.

The hydroxyl group introduced into the substrate can be converted to an alkoxy group in the routine manner, and the carboxyl group can be converted to an alkoxycarbonyl group, a carbamoyl group or an N-substituted carbamoyl group by utilizing the conventional esterification reaction or amidation reaction, for instance. Furthermore, the amino group can be converted to an N-substituted amino group using an alkylating agent or an acylating agent.

The compound and polymer having a basic group or an acidic group may form salts. For example, a carboxyl group-containing substrate can be reacted with an organic base or an inorganic base to give the corresponding salt. An amino group-containing substrate can be reacted with an inorganic acid or an organic acid to give the corresponding salt.

The oxidation and other reactions can be carried out in any of batch, semi-batch and continuous modes. After completion of each reaction, the reaction product can be easily isolated and purified by the conventional procedure.

### INDUSTRIAL APPLICABILITY

The acid-responsive compound of the present invention has an alicyclic hydrocarbon group (e.g. an adamantane skeleton) and becomes alkali-soluble upon exposure to light, with the result that it finds application as a photoresist in the formation of fine-line patterns. Furthermore, because it is high in sensitivity and etching resistance (dry etching resistance in particular), fine-line patterns can be formed with good reproducibility and high accuracy. In addition, it contributes to improved adhesion to the substrate and improved stability of the resist solution, thus insuring the formation of fine-line patterns with high accuracy and good reproducibility.

### EXAMPLES

Hereinafter, the present invention will be described in further detail with reference to Examples and Comparative Examples but should by no means be construed as defining the present invention.

### Example 1

### (1) Hydroxylation

To a solution of adamantan-1-yl-ethan-1-one (1 mol) in absolute tetrahydrofuran was added a solution of isopropylmagnesium iodide (iso-C₃H₇MgI) (1.2 mols) in absolute diethyl ether dropwise, and the mixture was stirred at 10°C for 6 hours to provide 1-(1-hydroxy-1,2-dimethylpropyl) adamantane.

### (2) Esterification

A mixture of 1-(1-hydroxy-1,2-dimethylpropyl)adamantane obtained above (1.00 mmol), samarium iodide (SmI₂) (0.10 mmol), isopropenyl acrylate (1.1 mmols) and dioxane (2 mmols) was stirred at 50°C for 6 hours. Analysis by gas chromatography revealed the formation of 1-(1-acryloyloxy-1,2-dimethylpropyl)adamantane of the following formula (yield 90%) in the reaction mixture.
Mass spectrum: [M] 276, 261, 218, 147, 135.

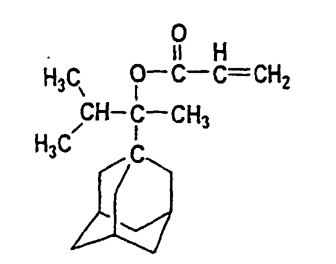

### (3) Polymerization

A monomer mixture (100 parts by weight) of the obtained 1-(1-acryloyloxy-1,2-dimethylpropyl)adamantane (50 weight %), methyl methacrylate (10 weight %), butyl acrylate (20 weight %) and methacrylic acid (20 weight %) was polymerized in an organic solvent (toluene) in the presence of polymerization initiator (benzoyl peroxide) (5 parts by weight), and the polymer was precipitated by adding methanol to the reaction mixture. The precipitate was purified by repeating the cycle of dissolution in toluene and precipitation with methanol to provide a copolymer having a weight average molecular weight of about 1.5×10⁴ (molecular weight in terms of polystylene according to GPC).

### Example 2

### (1)Hydroxylation

A mixture of 1-(1-acryloyloxy-1,2-dimethylpropyl)adamantane (10 mmols),NHPI(2 mmols), acetylacetonatocobalt(Co(AA)₂)(0.1 mmol) and acetic acid (25 ml) was stirred in an oxygen atmosphere at 75°C for 6 hours to provide 1-hydroxy-3-(1-acryloyloxy-1,2-dimethylpropyl)adamantane of the following formula (yield 78%).
Mass spectrum of hydroxyl-containing compound: [M] 292, 277, 233, 162, 145, 133.

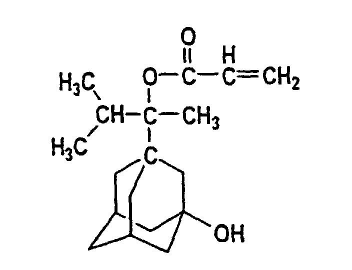

### (2) Polymerization

Using 1-hydroxy-3-(1-acryloyloxy-1,2-dimethylpropyl)adamantane in lieu of 1-(1-acryloyloxy-1,2-dimethylpropyl)adamantane, Step (3) of Example 1 was otherwise repeated to provide a copolymer.

### Example 3

### (1) Introduction of a carboxyl group

To acetic acid (25 ml) were added adamantan-1-yl-ethan-1-one (1-acetyladamantane) (10 mmols), NHPI (1 mmol) and Co(AA)₂ (0.005 mmol), and the mixture was stirred at 60°C for 6 hours in reactor with a gas pack inflated with a mixed gas (a mixed gas of carbon monoxide (2 L) and oxygen (0.5 L); pressure: 5 kg/cm²). As a result, 1-carboxyadamantan-3-yl-ethan-1-one was obtained at a conversion rate of 78% (yield 62%).

### (2) Hydroxylation

Using 1-carboxyadamantan-3-yl-ethan-1-one in lieu of adamantan-1-yl-ethan-1-one, Step (1) of Example 1 was otherwise repeated to provide 1-carboxy-3-(1-hydroxy-1,2-dimethylpropyl) adamantane (yield 60%).

### (3), (4) Esterification and polymerization

Using 1-carboxy-3-(1-hydroxy-1,2-dimethylpropyl)adamantane in lieu of 1-(1-hydroxy-1,2-dimethylpropyl)adamantane, Steps (2) and (3) of Example 1 were otherwise repeated to provide the carboxyl group-containing compound of the following formula, namely 1-carboxy-3-(1-acryloyloxy-1,2-dimethylpropyl)adamantane[i.e. 1-carboxy-3-(2-acryloyloxy-3-methyl-2-yl)adamantane] (yield 82%), and a copolymer.
Mass spectrum of carboxyl group-containing compound: [M] 320, 305, 262, 191, 146, 134.

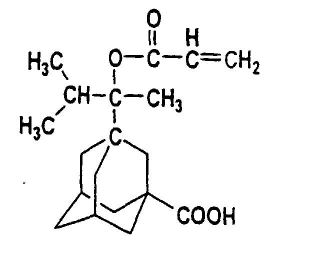

### Example 4

### (1) Hydroxylation

Using adamantanone in lieu of adamantan-1-yl-ethan-1-one, Step (1) of Example 1 was otherwise repeated to provide 2-isopropyl-2-hydroxyadamantane at the conversion rate of 76% (yield 61%).

### (2), (3) Esterification and polymerization

Using 2-isopropyl-2-hydroxyadamantane in lieu of 1-(1-hydroxy-1,2-dimethylpropyl) adamantane, Steps (2), (3) of Example 1 were otherwise repeated to provide the hydroxyl group-containing compound of the following formula, namely 2-isopropyl-2-acryloyloxyadamantane (yield 78%), and a copolymer.
Mass spectrum of hydroxyl group-containing compound: [M] 248, 233, 218, 205, 183, 139.

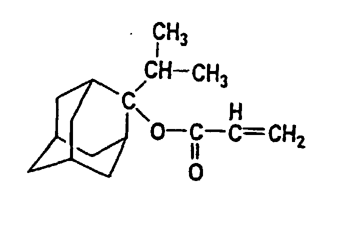

### Example 5

### (1) Hydroxylation

Using 2-isopropyl-2-acryloyloxyadamantane in lieu of 1-(1-acryloyloxy-1,2-dimethylpropyl)adamantane, the hydroxylation step (Step 2) of Example 2 was otherwise repeated to provide 1-hydroxy-4-isopropyl-4-acryloyloxyadamantane of the following formula (yield 56%).
Mass spectrum of hydroxyl group-containing compound: [M] 264, 246, 231, 216, 203, 176, 132.

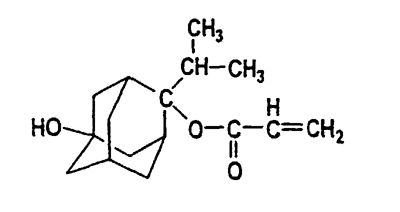

### (3) Polymerization

Using 1-hydroxy-4-isopropyl-4-acryloyloxyadamantane in lieu of 1-(1-acryloyloxy-1,2-dimethylpropyl)adamantane, Step (3) of Example 1 was otherwise repeated to provide a copolymer.

### Example 6

### (1) Introduction of a carboxyl group

Using adamantanone in lieu of adamantan-1-yl-ethan-1-one, the carboxylation step (Step 1) of Example 3 was otherwise repeated to provide 1-carboxyadamantan-4-one.

Using 1-carboxyadamantan-4-one in lieu of adamantan-1-yl-ethan-1-one, Step (1) of Example 1 was otherwise repeated to provide 1-carboxy-4-hydroxy-4-isopropyladamantane (yield 58%).

### (2), (3) Esterification and polymerization

Using 1-carboxy-4-hydroxy-4-isopropyladamantane in lieu of 1-(1-hydroxy-1,2-dimethylpropyl)adamantane, Steps (2) and (3) of Example 1 were otherwise repeated to provide the carboxyl group-containing compound of the following formula, namely 1-carboxy-4-acryloyloxy-4-isopropyladamantane (yield 81%), and a copolymer.
Mass spectrum of carboxyl group-containing compound: [M] 292, 221, 206, 191, 178, 133.

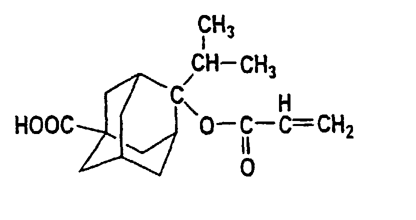

### Example 7

### (1) Hydroxylation

A mixture of 1-acetyladamantane (10 mmols), NHPI (2 mmols), acetylacetonatocobalt (Co(AA)₂) (0.1 mmol) and acetic acid (25 ml) was stirred in an oxygen atmosphere at 75°C for 6 hours, whereby 1-hydroxy-3-acetyladamantane was obtained (yield 80%).

### (2) Reduction

A mixture of 1-hydroxy-3-acetyladamantane (2 mmols), sodium boron hydride (NaBH₄) (2.4 mmols) and tetrahydrofuran (25 ml) was stirred at room temperature for 3 hours. As a result, 1-hydroxy-3-(1-hydroxyethyl)adamantane was obtained (yield 95%).

### (3) Esterification and polymerization

A mixture of 1-hydroxy-3-(1-hydroxyethyl)adamantane (1 mmol), acryloyl chloride (1.2 mols), triethylamine (1.2 mols) and dioxane (10 ml) was stirred at 40°C for 3 hours. As a result, 1-hydroxy-3-(1-acryloyloxyethyl)adamantane of the following formula was obtained (yield 78%).

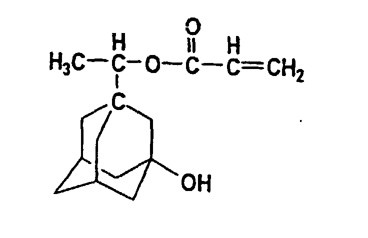

Using 1-hydroxy-3-(1-acryloyloxyethyl)adamantane in lieu of 1-(1-acryloyloxy-1,2-dimethylpropyl)adamantane, the polymerization step of Example 1 was otherwise repeated to provide a copolymer.

### Example 8

### (1) Hydroxylation

To a solution of adamantanecarbonyl chloride (1 mol) in absolute tetrahydrofuran was added a solution of ethylmagnesium iodide (C₂H₅MgI) (2.2 mols) in absolute diethyl ether dropwise, and the mixture was stirred at room temperature for 6 hours to provide 1-(3-hydroxypent-3-yl)adamantane (yield 95%).
Mass spectrum: [M] 222, 204, 193, 175, 161, 147, 135.

A mixture of 1-(3-hydroxypent-3-yl)adamantane (10 mmols), NHPI (2 mmols), acetylacetonatocobalt (Co(AA)₂) (0.1 mmol) and acetic acid (25 ml) was stirred in an oxygen atmosphere at 75°C for 6 hours. As a result, 1-hydroxy-3-(3-hydroxypent-3-yl)adamantane was obtained (yield 80%).

### (2) Esterification and polymerization

A mixture of 1-hydroxy-3-(3-hydroxypent-3-yl)adamantane (1 mmol), acryloyl chloride (1.2 mmols), triethylamine (1.2 mols) and dioxane (10 ml) was stirred at 40°C for 3 hours. As a result, 1-hydroxy-3-(3-acryloyloxypent-3-yl)adamantane of the following formula was obtained (yield 45%).

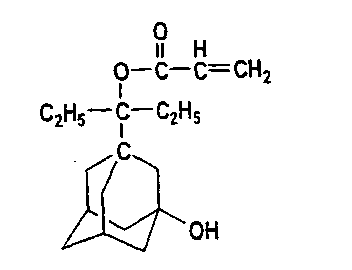

Using 1-hydroxy-3-(3-acryloyloxypent-3-yl)adamantane in lieu of 1-(1-acryloyloxy-1,2-dimethylpropyl)adamantane, the polymerization step of Example 1 was otherwise repeated to provide a copolymer.

### Example 9

### (1) Hydroxylation

To a solution of adamantanecarbonyl chloride (1 mol) in absolute tetrahydrofuran was added a solution of ethylmagnesium iodide (C₂H₅MgI) (1.1 mol) in absolute diethyl ether dropwise, and the mixture was stirred at room temperature for 6 hours to provide 1-(1-hydroxyproyl)adamantane (yield 80%).
Mass spectrum: [M] 194, 176, 165, 147, 135.

A mixture of 1-(1-hydroxypropyl)adamantane (10 mmols), NHPI (2 mmols), acetylacetonatocobalt (Co(AA)₂) (0.1 mmol) and acetic acid (25 ml) was stirred in an oxygen atmosphere at 75°C for 6 hours. As a result, 1-hydroxy-3-(1-oxopropyl)adamantane was obtained (yield 80%).

A mixture of 1-hydroxy-3-(1-oxopropyl)adamantane (2 mmols), sodium boron hydride (NaBH₄) (2.4 mmols) and tetrahydrofuran (25 ml) was stirred at room temperature for 3 hours. As a result, 1-hydroxy-3-(1-hydroxypropyl)adamantane was obtained (yield 95%).

### (2) Esterification and polymerization

A mixture of 1-hydroxy-3-(1-hydroxypropyl)adamantane (1 mmol), acryloyl chloride (1.2 mols), triethylamine (1.2 mols) and dioxane (10 ml) was stirred at 40°C for 3 hours. As a result, 1-hydroxy-3-(1-acryloyloxypropyl)adamantane of the following formula was obtained (yield 75%).

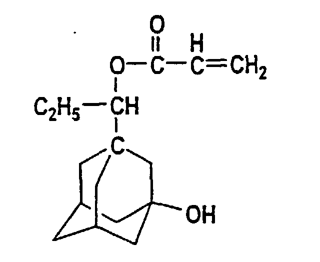

Using 1-hydroxy-3-(1-acryloyloxypropyl)adamantane in lieu of 1-(1-acryloyloxy-1,2-dimethylpropyl)adamantane, the polymerization step of Example 1 was otherwise repeated to provide a copolymer.

### Example 10

### (1) Hydroxylation

A mixture of adamantane-1-carboxylic acid (10 mmols), NHPI (2 mmols), acetylacetonatocobalt (Co(AA)₂) (0.1 mmol) and acetic acid (25 ml) was stirred in an oxygen atmosphere at 75°C for 6 hours. As a result, 1-hydroxy-3-carboxyadamantane was obtained (yield 80%).

### (2) Reduction

A mixture of 1-hydroxy-3-carboxyadamantane (2 mmols), sodium boron hydride (NaBH₄) (6 mmols) and tetrahydrofuran (25 ml) was stirred at room temperature for 6 hours. As a result, 1-hydroxy-3-hydroxymethyladamantane was obtained (yield 90%).

### (3) Esterification and polymerization

A mixture of 1-hydroxy-3-hydroxymethyladamantane (1 mmol), acryloyl chloride (1.2 mols), triethylamine (1.2 mols) and dioxane (10 ml) was stirred at 40°C for 3 hours. As a result, 1-hydroxy-3-(acryloyloxymethyl)adamantane of the following formula was obtained (yield 90%).

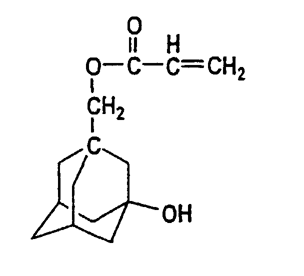

Using 1-hydroxy-3-(acryloyloxymethyl)adamantane in lieu of 1-(1-acryloyloxy-1,2-dimethylpropyl)adamantane, the polymerization step of Example 1 was otherwise repeated to provide a copolymer.

### Example 11

### (1) Hydroxylation

A mixture of decalin (10 mmols), NHPI (2 mmols), acetylacetonatocobalt (Co(AA)₂) (0.1 mmol) and acetic acid (25 ml) was stirred in an oxygen atmosphere at 75°C for 6 hours. As a result, 9,10-dihydroxy-bicyclo[4.4.0]decane was obtained (yield 70%).

### (2) Esterification and polymerization

A mixture of 9,10-dihydroxy-bicyclo[4.4.0]decane (1 mmol), acryloyl chloride (1.2 mols), triethylamine (1.2 mols) and dioxane (10 ml) was stirred at 40°C for 3 hours. As a result, 9-hydroxy-10-acryloyloxy-bicyclo[4.4.0]decane of the following formula was obtained (yield 90%).

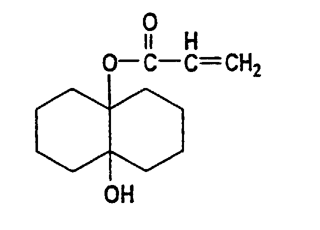

Using 9-hydroxy-10-acryloyloxy-bicyclo[4.4.0]decane in lieu of 1-(1-acryloyloxy-1,2-dimethylpropyl)adamantane, the polymerization step of Example 1 was otherwise repeated to provide a copolymer.

### Example 12

### (1) Hydroxylation

A mixture of tricyclo [5.2.1.0^{2,6}]decane (10 mmols), NHPI (2 mmols), acetylacetonatocobalt (Co(AA)₂) (0.1 mmol) and acetic acid (25 ml) was stirred in an oxygen atmosphere at 75°C for 6 hours. As a result, 2,6-dihydroxytricyclo[5.2.1.0^{2,6}]decane was obtained (yield 70%).

### (2) Esterification and polymerization

A mixture of 2,6-dihydroxy-tricyclo[5.2.1.0^{2,6}]decane (1 mmol), acryloyl chloride (1.2 mols),triethylamine (1.2 mols) and dioxane (10 ml) was stirred at 40°C for 3 hours. As a result, 2-hydroxy-6-acryloyloxy-tricyclo[5.2.1.0^{2,6}]decane of the following formula was obtained (yield 70%).

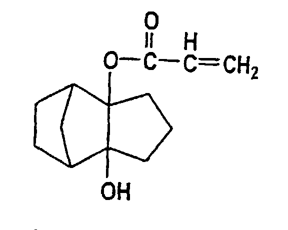

Using 2-hydroxy-6-acryloyloxy-tricyclo[5.2.1.0^{2,6}]decane in lieu of 1-(1-acryloyloxy-1,2-dimethylpropyl)adamantane, the polymerization step of Example 1 was otherwise repeated to provide a copolymer.

### Example 13

### (1) Hydroxylation

To a solution of adamantanecarbonyl chloride (1 mol) in absolute tetrahydrofuran was added a solution of ethylmagnesium iodide (C₂H₅MgI) (2.2 mols) in absolute diethyl ether dropwise. and the mixture was stirred at room temperature for 6 hours. As a result, 1-(3-hydroxypent-3-yl)adamantane was obtained (yield 95%).
Mass spectrum: [M] 222, 204, 193, 175, 161, 147, 135.

A mixture of 1-(3-hydroxypent-3-yl)adamantane (10 mmols), NHPI (2 mmols), acetylacetonatocobalt (Co(AA)₂)(0.1mmol)and acetic acid (25 ml) was stirred in an oxygen atmosphere at 85°C for 10 hours. As a result, 1,3-dihydroxy-5-(3-hydroxypent-3-yl)adamantane was obtained (yield 50%).

### (2) Esterification and polymerization

A mixture of 1,3-dihydroxy-5-(3-hydroxypent-3-yl)adamantane (1 mmol), acryloyl chloride (0.5 mmol), triethylamine (0.5 mmol) and dioxane (10 ml) was stirred at 40°C for 3 hours. As a result, 1,3-dihydroxy-5-(3-acryloyloxypent-3-yl)adamantane of the following formula was obtained (yield 80% based on acryloyl chloride).

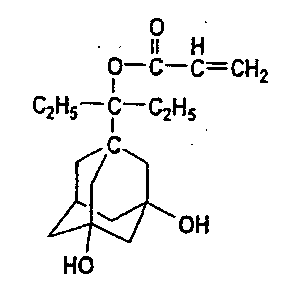

Using 1,3-dihydroxy-5-(3-acryloyloxypent-3-yl)adamantane in lieu of 1-(1-acryloyloxy-1,2-dimethylpropyl)adamantane, the polymerization step of Example 1 was otherwise repeated to provide a polymer.

### [Photoresist resin composition]

A photoresist resin composition was prepared by mixing 100 parts by weight of each polymer obtained and 15 parts by weight of triphenylphosphoniumhexafluoroantimony with the solvent toluene. This photoresist resin composition was coated on a silicon wafer by the spin coating technique to form a 1.0 µm thick photosensitive layer. After prebaking on a hot plate at 60°C for 100 seconds, exposure was carried out at an illuminating amount 100 mJ/cm² using a KrF excimer stepper, followed by postbaking at 100°C for 60 seconds. Development was then carried out with an aqueous alkaline solution (Tokyo Oka K.K., NMD-3) for 60 seconds and the wafer was rinsed with pure water. As a result, a predetermined pattern (a line-and-space pattern of 0.3 µm in width each) could be formed.

## Claims

1. An acid-responsive compound represented by the following formula (1) or (2) 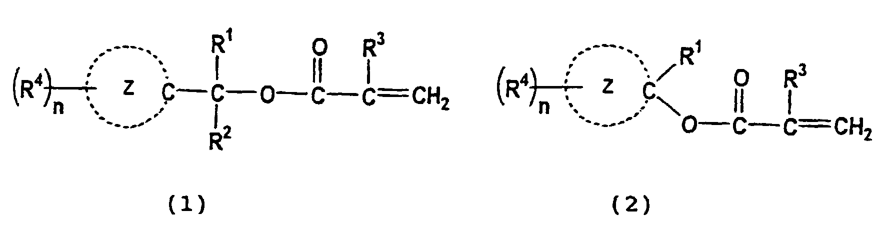 wherein R¹ and R² are the same or different from each other and each represents a hydrogen atom, an alkyl group or a cycloalkyl group; R³ represents a hydrogen atom or a methyl group; R⁴ represents a hydrogen atom, a halogen atom, an alkyl group, an oxygen-containing group, an amino group or an N-substituted amino group; n represents an integer of not less than 1; with proviso that all R⁴s are not concurrently hydrogen atoms, and R⁴ may be vary according to n; the Z ring represents a monocyclic or polycyclic alicyclic hydrocarbon ring; in formula (1), R¹ and R² may, jointly and together with the adjacent carbon atom, form an alicyclic hydrocarbon ring.

2. The acid-responsive compound according to Claim 1 wherein, in the formula (1), R¹ is a hydrogen atom and R² is a hydrogen atom or a straight-chain or branched-chain C₁₋₄ alkyl group.

3. The acid-responsive compound according to Claim 1 wherein the Z ring is a bridged ring-type hydrocarbon ring comprising 2 to 4 rings.

4. The acid-responsive compound according to Claim 1 wherein the oxygen-containing group is at least one substituent selected from the group consisting of oxo group, hydroxyl group, an alkoxy group, carboxyl group, an alkoxycarbonyl group, a cycloalkyloxycarbonyl group, an aryloxycarbonyl group, an aralkyloxycarbonyl group, hydroxymethyl group, carbamoyl group, an N-substituted carbamoyl group and nitro group.

5. The acid-responsive compound according to Claim 1 wherein R⁴ is a hydroxyl group, an alkoxy group, a carboxyl group, an alkoxycarbonyl group or a hydroxymethyl group .

6. The acid-responsive compound according to Claim 1 wherein R⁴ is a hydroxyl group or a carboxyl group and n is an integer of 2 to 4.

7. The acid-responsive compound according to Claim 1, which is represented by the following formula (1a) or (2a): 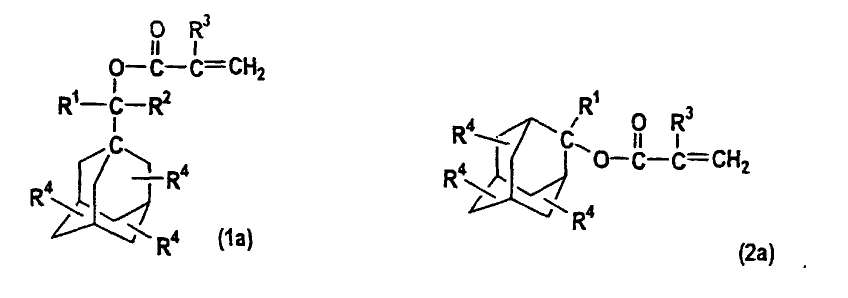 wherein R¹, R² and R³ are as defined above; R⁴s may be the same or different from each other and each represents a hydrogen atom, a halogen atom, an alkyl group, an oxygen-containing group, an amino group or an N-substituted amino group; with proviso that all R⁴s are not concurrently hydrogen atoms.

8. The acid-responsive compound according to Claim 7 wherein R¹ is a hydrogen atom or a straight-chain or branched-chain C₁₋₄ alkyl group; R² is a hydrogen atom or a straight-chain or branched-chain C₁₋₄ alkyl group; R³ is a hydrogen atom or a methyl group; at least one of R⁴s is at least one oxygen-containing group selected from the group consisting of oxo group, hydroxyl group, an alkoxy group, carboxyl group, an alkoxycarbonyl group, a cycloalkyloxycarbonyl group, an aryloxycarbonyl group, an aralkyloxycarbonyl group, hydroxymethyl group, carbamoyl group, an N-substituted carbamoyl group and nitro group.

9. The acid-responsive compound according to Claim 1 which is represented by the following formula (2d) or (2e); 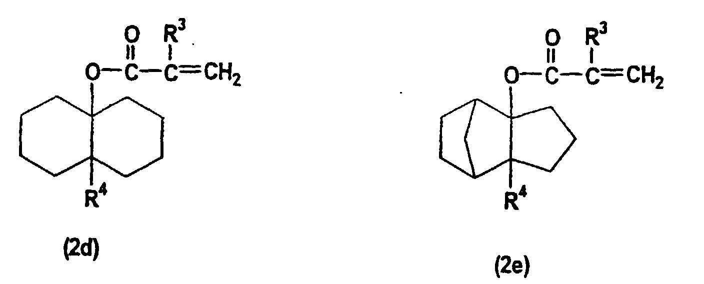 wherein R³ and R⁴ are as defined above.

10. A photoresist resin composition comprising a polymer having at least a unit represented by the following formula (11) or (12); 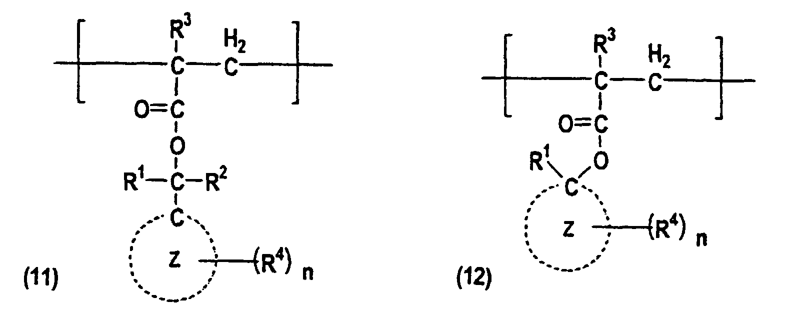 wherein R¹, R², R³, R⁴, Z ring and n are as defined in Claim 1
and a photoactive acid precursor.

11. The photoresist resin composition according to Claim 10 wherein the Z ring is an adamantane ring.

12. The photoresist resin composition according to Claim 10 which contains 0.1 to 30 parts by weight of the photoactive acid precursor relative to 100 parts by weight of the polymer.

13. The photoresist resin composition according to Claim 8 wherein the polymer is a copolymer.

14. A method of forming a pattern which comprises subjecting a layer comprising the photoresist resin composition of Claim 8 formed on a substrate to pattern exposure and
developing the exposed coating layer to form a pattern.
